# EUROPÄISCHE PATENTSCHRIFT

(11) **EP 3 452 446 B1**
(45) Veröffentlichungstag und Bekanntmachung des Hinweises auf die Patenterteilung: **28.06.2023**
(21) Anmeldenummer: 17721580.3
(22) Anmeldetag: 26.04.2017
(51) Int. Cl.: C07D 201/02, C07D 207/38

(54) **VERFAHREN ZUR HERSTELLUNG VON CIS-ALKOXYSUBSTITUIERTEN SPIROCYCLISCHEN 1-H-PYRROLIDIN-2,4-DION- DERIVATEN**
PROCESS FOR THE PREPARATION CIS-ALKOXYSUBSTITUTED SPIROCYCLIC 1-H-PYRROLIDIN-2,4-DIONE DERIVATIVES
PROCÉDÉ POUR LA PREPARATION DES DERIVÉS DE SPIROCYCLIQUE 1-H-PYRROLIDIN-2,4-DIONE SUBSTITUÉS PAR CIS-ALKOXY

(30) Priorität: 04.05.2016 EP 16168243
(43) Veröffentlichungstag der Anmeldung: 13.03.2019
(73) Patentinhaber: Bayer CropScience Aktiengesellschaft, 40789 Monheim am Rhein (DE)
(72) Erfinder: FARIDA, Taraneh, 50259 Pulheim-Geyen (DE); LITTMANN, Martin, 51375 Leverkusen (DE); WARSITZ, Rafael, 45136 Essen (DE); ESSER, Michael, 51379 Leverkusen (DE); SCHNATTERER, Albert, 51373 Leverkusen (DE)
(74) Vertreter: BIP Patents
(86) Internationale Anmeldenummer: PCT/EP2017/059885
(87) Internationale Veröffentlichungsnummer: WO 2017/191001

(56) Entgegenhaltungen:
- WO-A1-2004/007448
- WO-A2-2006/024411

## Beschreibung

Die vorliegende Erfindung betrifft neue Verfahren zur Herstellung von cis-alkoxysubstituierten spirocyclischen 1-H-Pyrrolidin-2,4-dion-Derivaten sowie neue Intermediate bzw. Ausgangsverbindungen, welche in dem erfindungsgemäßen Verfahren durchlaufen bzw. verwendet werden.

Bekannt ist die mehrstufige Reaktion von alkoxysubstituierten spirocyclischen 1-H-Pyrrolidin-2,4-dion-Derivaten (WO 98/05638, WO 04/007448).

Nachteilig an dem bisherigen Verfahren ist, dass Zwischenverbindungen isoliert werden müssen. Die Isolierung von Zwischenverbindungen macht das Verfahren technisch sehr aufwändig und führt zu Ausbeuteverlusten. Durch die Aufarbeitung fällt Abwasser an, das nur sehr aufwändig entsorgt werden kann.

Die Aufgabe der vorliegenden Erfindung besteht in der Bereitstellung neuer, wirtschaftlich und ökologisch günstigeren Verfahren zur Herstellung von Verbindungen der Formel (I).

Überraschenderweise können nach dem erfindungsgemäßen Verfahren cis-alkoxysubstituierte spirocyclische 1-H-Pyrrolidin-2,4-dion-Derivate (Verbindungen der Formel (I)) auf einfachere Weise, in einem Eintopfverfahren, ohne Isolierung der Zwischenverbindungen, in höherer Reinheit und in besserer Ausbeute hergestellt werden ausgehend von Verbindungen der Formel (II). Durch den Einsatz des Eintopfverfahrens kann zudem die Menge an Base (Säurebindemittel) verringert und die Abwassermenge reduziert werden.

Es wurde nun gefunden, dass man in einer Eintopfreaktion Verbindungen der Formel (I) 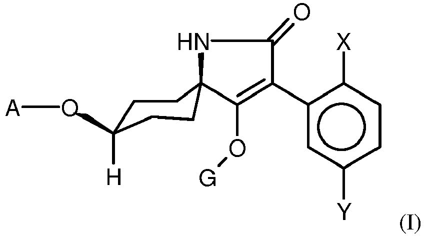 in welcher
- X: für Methyl steht,
- Y: für Methyl steht,
- A: für Methyl steht,
- G: für die Gruppe 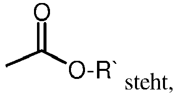
worin
- R': für Ethyl steht,
erhält,
in dem man zunächst Verbindungen der Formel (II) 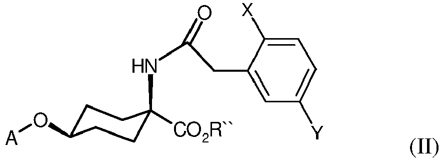 in welcher X, Y und A die oben genannten Bedeutungen haben und
- R": für Methyl steht,
in Gegenwart von Natriummethylat und in Gegenwart von DMAC zu Verbindungen der Formel (III) 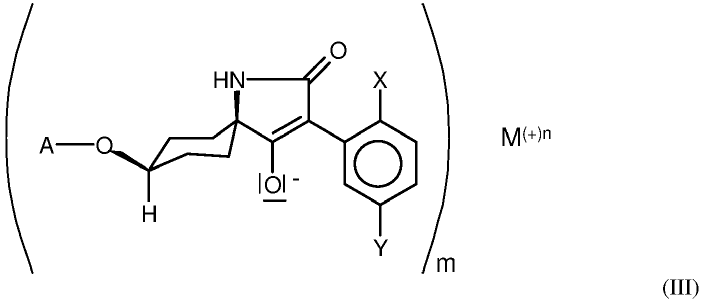 in welcher X, Y und A die oben genannten Bedeutungen haben und
M für Natrium steht,
m für die Zahl 1 steht,
n für die Zahl 1 steht
cyclisiert
und mit Verbindungen der Formel (IV) 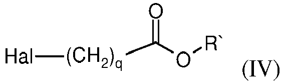
in welcher
- R': für Ethyl steht,
- q: für die Zahl 0 oder 1 steht,
und Hal für Chlor steht,
gegebenenfalls in Gegenwart von DMAC und gegebenenfalls in Gegenwart von Trietyhlamin als Säurebindemittel und gegebenenfalls in Anwesenheit von Aliquat 336 als Phasentransferkatalysator umsetzt.

Im o.g. Verfahren kann es zu einer Rückspaltung von Verbindungen der Formel (I) zu Verbindungen der Formel (III) kommen, die durch Umsetzung mit Verbindungen der Formel (IV) 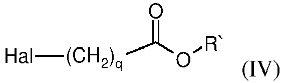
in welcher R' und q die oben angegebenen Bedeutungen haben
und Hal für Chlor steht,
gegebenenfalls in Gegenwart von Xylol und gegebenenfalls in Gegenwart von Triethylamin als Säurebindemittel
zu Verbindungen der Formel (I) rückgeführt werden (Rückführung).

Darüberhinaus kann die Eintopfreaktion auch in DMAC (Dimethylacetamid) - freien Lösungsmitteln erfolgen, was eine weitere Verfahrensverbesserung darstellt, da die Entsorgung von DMAC-haltigen Abwasser mit hohen Kosten verbunden ist. Insbesondere soll dieses Verfahren mit gängigen Lösungsmitteln auskommen. Besonders bevorzugt wird Xylol verwendet.

### Insbesonders bevorzugt ist die Verbindung der Formel (I-1)

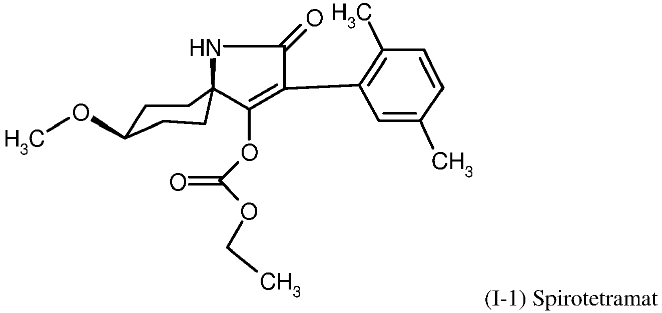

### Insbesonders bevorzugt ist die Verbindung der Formel (II-1)

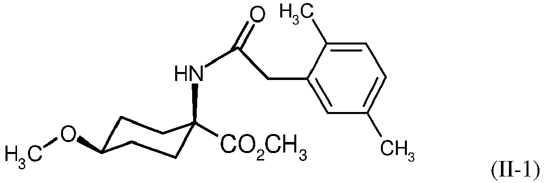

### Insbesonders bevorzugt ist die Verbindung der Formel (III-1)

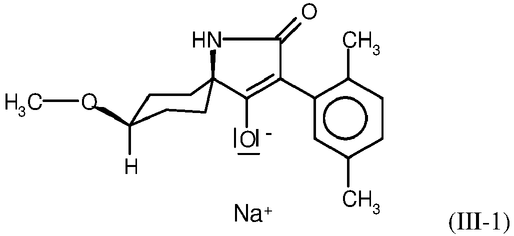

Die Verbindungen der Formel (I) sind bekannt (WO 98/05638, WO 04/007448) oder lassen sich nach den dort beschriebenen Verfahren herstellen.

Die Verbindungen der Formel (II) sind bekannt (WO 98/05638, WO 04/007448, WO 13/144101) oder lassen sich nach den dort beschriebenen Verfahren herstellen.

WO 2006/024411 offenbart mit der Verbindung A.11 strukturell ähnliche Verbindungen wie die Verbindungen der Formel (III).

Die Verbindungen der Formel (IV) sind kommerziell erhältlich.

### Schema 1: Eintopfreaktion mit Rückführung

Der Verlauf des erfindungsgemäßen Verfahrens wird durch folgendes Reaktionsschema wiedergegeben:

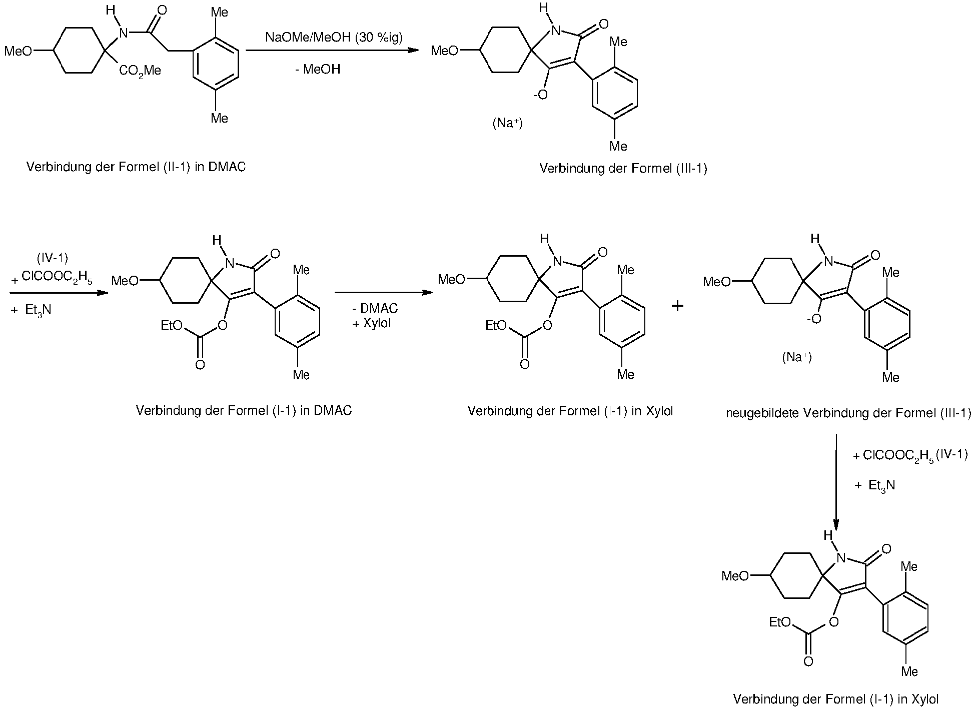

Bedingt durch das cis/trans Isomerenverhältnis der Verbindungen der Formel (II), die in das Herstellverfahren eingesetzt werden, fallen die Verbindungen der Formeln (I) und (III) in Form cis/trans Isomerengemische an, wobei in dem erfindungsgemäßen Verfahren hauptsächlich cis-Isomer entsteht.

Das Verfahren ist dadurch gekennzeichnet, dass man Verbindungen der Formel (II) mit hohem cis-Isomerenanteil in Gegenwart von Natriummethylat und in Gegenwart von Lösungsmitteln zu den entsprechenden Verbindungen der Formel (III) cyclisiert. Die Verbindungen der Formel (III) werden anschließend mit Verbindungen der Formel (IV) zu Verbindungen der Formel (I) gegebenenfalls in Gegenwart von Lösungsmitteln und gegebenenfalls in Gegenwart von Triethylamin als Säurebindemittel und gegebenenfalls in Anwesenheit von Aliquat 336 als Phasentransferkatalysator umgesetzt.

Die Reaktionstemperatur zur Herstellung der Verbindungen der Formel (III) kann bei der Durchführung des erfindungsgemäßen Verfahrens variiert werden. Im Allgemeinen arbeitet man bei Temperaturen zwischen 20 °C und 110 °C, vorzugsweise zwischen 60 °C und 90 °C.

Als Base können Alkoholate sowohl als Feststoff als auch als Lösung eingesetzt werden. Z. Bsp. NaOMe fest oder als Lösung in Methanol. Bevorzugt ist festes Natriummethylat oder Natriummethylat in Methanol 30 %ig. Besonders bevorzugt ist Natriummethylat in Methanol 30 %ig.

Als Lösungsmittel können DMAC oder Xylol verwendet werden. Besonders bevorzugt verwendet wird DMAC.

Die Reaktionstemperatur zur Herstellung der Verbindungen der Formel (I) kann bei der Durchführung des erfindungsgemäßen Verfahrens variiert werden. Im Allgemeinen arbeitet man bei Temperaturen zwischen 20 °C und 100 °C, bevorzugt zwischen 50 °C und 70 °C.

Als Säurebindemittel kommen alle üblichen Säureakzeptoren in Betracht. Bevorzugt verwendet wird Triethylamin.

Als Phasentransferkatalysator kann Aliquat 336 eingesetzt werden.

Bei der Durchführung des erfindungsgemäßen Verfahrens setzt man die Reaktionskomponenten der Formel (II) im Allgemeinen in äquimolaren bis etwa doppeltäquimolaren Mengen ein.

In einem bevorzugten Verfahren werden die Verbindungen der Formel (II) mit NaOCH₃ (fest oder als Lösung in Methanol) in DMAC zum Natriumsalz der Verbindungen der Formel (III) umgesetzt. Entstehendes Methanol muss zur Vermeidung von Nebenkomponenten auf der Folgestufe abdestilliert werden. Die nachfolgende Umsetzung mit dem Säurechlorid der Formel (IV) erfolgt dann unter katalytischem Säurebindemittelzusatz (z.B. Triethylamin). Anschließend kann das Lösungsmittel im Vakuum nahezu vollständig abdestilliert werden. Je nach Destillationsbedingungen kann es dabei zur Rückspaltung von Verbindungen der Formel (I) zu Verbindungen der Formel (III) kommen.

In einem ganz bevorzugten Verfahren wird nach der Lösungsmitteldestillation der Sumpf mit einer geringen Menge an Säurebindemittel versetzt (z.B. Trietyhlamin) und mit Säurechlorid der Formel (IV) zu Verbindungen der Formel (I) rückgeführt. Mit dieser Verfahrensvariante werden ausgehend von Verbindungen der Formel (II) nahezu vollständige Ausbeuten an Verbindungen der Formel (I) erzielt (>95%)

Die Umsetzung von Verbindungen der Formel (II) in Gegenwart von Natriummethylat und in Gegenwart von Lösungsmitteln zu Verbindungen der Formel (III) und anschließender Umsetzung mit Verbindungen der Formel (IV) gegebenenfalls in Gegenwart von Lösungsmitteln und gegebenenfalls in Gegenwart von Triethylamin als Säurebindemittel und gegebenenfalls in Anwesenheit von Aliquat 336 als Phasentransferkatalysator zu Verbindungen der Formel (I) lässt sich überraschenderweise auch in unpolaren Lösungsmitteln (wie z.Bsp. Toluol, Xylol, Alkane wie n-Hexan, n-Heptan, n-Octan, Kohlenwasserstoffe wie Pentan, Hexan, Heptan, Cyclohexan, Methylcyclohexan, Benzol, halogenierte Kohlenwasserstoffe wie Methylenchlorid, Chloroform, Tetrachlorkohlenstoff, Chlorbenzol, o-Dichlorbenzol, Dichlorethan; besonders bevorzugt verwendet wird Xylol) durchführen, wenn die Umsetzung von Verbindungen der Formel (III) mit Verbindungen der Formel (IV) in Gegenwart von Wasser durchgeführt wird. Dabei ist in der Acylierungsstufe (Umsetzung mit Verbindungen der Formel (IV)) auf einen geeigneten pH-Wert (bevorzugt zwischen pH 3 und 12, besonders bevorzugt zwischen pH 8 und 10) zu achten, bei dem die Reaktivität der Verbindungen der Formel (III) noch ausreichend hoch ist, gleichzeitig aber die Stabilität des Produktes der Formel (I) und des Säurechlorides der Verbindungen der Formel (IV) gegenüber alkalischer Verseifung gewahrt bleibt. Die Einstellung des pH-Wertes kann z.B. durch kontinuierliche Zugabe einer Base (z.B. MOH oder M(OH)₂ oder M(OH)₃, wobei M die o.g. Bedeutung hat) erfolgen. Bevorzugt ist wässrige Natronlauge. Die Umsetzung kann durch Zugabe katalytischer Mengen eines Phasentransferkatalysators unterstützt werden. In einer bevorzugten Verfahrensvariante kann das Säurebindemittel komplett oder teilweise durch einen Phasentransferkatalysator ersetzt werden. Die Umsetzung der Verbindungen der Formel (II) zu Verbindungen der Formel (III) kann durch die Zugabe von Co-Lösungsmitteln unterstützt werden. Als Co-Lösungsmittel können Alkohole, wie Methanol oder Ethanol verwendet werden, bevorzugt wird Methanol verwendet.

### Herstellungsbeispiele:

### Beispiel 1: Eintopfverfahren in DMAC (ohne Rückführung nach der DMAC Destillation) zur Herstellung von Verbindung der Formel (I-1)

Es werden 807,69 g einer Lösung der Verbindung der Formel (II-1) (0,81 mol) in DMAC vorgelegt. Bei einer Innentemperatur von 60 - 65 °C werden 159,04 g einer 30%igen Natriummethylatlösung in Methanol (0,88 mol) in ca. 2,5 h zudosiert. Anschließend wird Methanol im Vakuum abdestilliert. Die Reaktionslösung wird auf 50 °C abgekühlt, 9,62 g Triethylamin werden zudosiert. Anschließend werden während ca. 2,5 Stunden 101,54 g Chlorameisensäureethylester (0,94 mol) zwischen 52 und 56°C zudosiert. Es wird noch eine halbe Stunde nachgerührt, dann wird ein Teil DMAC abdestilliert.

Anschließend wird das restliche DMAC vollständig abdestilliert.

Nach Zugabe von 403,85 g Xylol wird der Reaktorinhalt auf 56°C abgekühlt.

Anschließend wird die Xylol-Lösung auf ca. 80°C erwärmt und 173,08 g einer 1,6%igen Natriumhydrogencarbonat-Lösung zugegeben und die wässrige Phase abgetrennt. Anschließend wird noch zweimal mit jeweils 107,69 g Wasser gewaschen.

Die gewaschene Xylol-Phase wird im Vakuum destilliert und aufkonzentriert (307,69 g Xylol). Anschließend werden 148,08 g Methylcyclohexan zugegeben und es wird von ca. 78°C auf 23°C abgekühlt.

Die Suspension wird bei 23-25 °C filtriert. Der feuchte Filterkuchen wird mit Methylcyclohexan gewaschen und getrocknet.

Die Ausbeute beträgt 90 % der Theorie.

### Beispiel 2: Eintopfverfahren in DMAC (mit Rückführung nach der DMAC Destillation) zur Herstellung von Verbindung der Formel (I-1)

Es werden 807,69 g einer Lösung der Verbindung der Formel (II-1) (0,81 mol) in DMAC vorgelegt. Bei einer Innentemperatur von 60 - 65 °C werden 159,04 g einer 30%igen Natriummethylatlösung in Methanol (0,88 mol) in ca. 2,5 h zudosiert. Anschließend wird Methanol im Vakuum abdestilliert. Die Reaktionslösung wird auf 50 °C abgekühlt, 9,62 g Triethylamin werden zudosiert. Anschließend werden während ca. 2,5 Stunden 101,54 g Chlorameisensäureethylester (0,94 mol) zwischen 52 und 56°C zudosiert. Es wird noch eine halbe Stunde nachgerührt, dann wird ein Teil DMAC abdestilliert.

Anschließend wird das restliche DMAC vollständig abdestilliert.

Nach Zugabe von 403,85 g Xylol wird der Reaktorinhalt auf 56°C abgekühlt.

Rückführung: Es werden 3,85 g Triethylamin und 9,62 g Chlorameisensäureethylester (0,09 mol) nachdosiert.

Anschließend wird die Xylol-Lösung auf ca. 80°C erwärmt und 173,08 g einer 1,6%igen Natriumhydrogencarbonat-Lösung zugegeben und die wässrige Phase abgetrennt. Anschließend wird noch zweimal mit jeweils 107,69 g Wasser gewaschen.

Die gewaschene Xylol-Phase wird im Vakuum destilliert und aufkonzentriert (307,69 g Xylol). Anschließend werden 148,08 g Methylcyclohexan zugegeben und es wird von ca. 78°C auf 23°C abgekühlt.

Die Suspension wird bei 23-25 °C filtriert. Der feuchte Filterkuchen wird mit Methylcyclohexan gewaschen und getrocknet.

Die Ausbeute beträgt 95 % der Theorie.

### Beispiel 3: Herstellung von Verbindung der Formel (I-1)

537,8 g der Verbindung der Formel (II-1) in Xylol/Methanol (0,500 mol, ca. 31 %ig) wird vorgelegt. Methanol wird bei Normaldruck abdestilliert. 105 g Na-Methanolat in Methanol (0,583 mol, 30 %ig) werden in 1 h bei 85 °C dosiert. Während der Na-Methanolat Dosierung wird Methanol bei 85 °C abdestilliert. Nach Ende der Dosierung wird 4 h bei 85 °C nachgerührt. Um die Temperatur von 85 °C zu halten, wird zwischendurch Methanol destilliert. Nach 4 h ist der Umsatz vollständig. Das Reaktionsgemisch wird für die Umsetzung auf 80 °C abgekühlt.

118,1 g HCl 2 % ig (0,06 mol) werden bei Raumtemperatur vorgelegt. Zu dieser Vorlage werden 437,8 g der Verbindung der Formel (III-1) in Xylol/Methanol (34 %ig, 0,495 mol) gegeben. Das Gemisch wird anschließend im Vakuum methanolfrei destilliert. Es wird auf 50 °C abgekühlt und 150 g Xylol und 15,3 g Triethylamin (0,150 mol) zugegeben. Anschließend werden 76,74 g Chloressigsäureethylester (0,700 mol, 99 %ig) in 2 h bei 50 °C dosiert. Durch die Paralleldosierung von Natronlauge 32 %ig wird der pH-Wert zwischen 9,5-10 gehalten. Es wird ½ h bei 50 °C nachgerührt. Mit einer 18 %igen Salzsäurelösung wird ein pH-Wert von 2 eingestellt und Wasser zugegeben. Für die Phasentrennung wird auf 75 °C aufgeheizt, wobei der pH-Wert mit Natronlauge nachgestellt wird und anschließend die Phasen getrennt. Es werden 150 g Natriumhydrogencarbonat-Lösung 2 %ig bei 75 °C zugegeben und die Phasen getrennt. Der pH-Wert liegt bei ca. 8. Die organische Phase wird bei ca. 75 mbar und 60-70 °C entwässert und aufkonzentriert. 90,6 g Methylcyclohexan werden bei 75 °C zugegeben, wobei Feststoff (Verbindung der Formel (I-1)) ausfällt. Das Reaktionsgemisch wird auf 109 °C (Rückfluss) aufgeheizt, auf 20 °C abgekühlt und isoliert. Der Feststoff (Verbindung der Formel (I-1)) wird durch eine Verdrängungswäsche mit MCH gewaschen und anschließend getrocknet. Die isolierte Ausbeute beträgt 92-93 % d. Th. bezogen auf die Verbindung der Formel (II-1).

### Beispiel 4: Herstellung von Verbindung der Formel (I-1)

537,8 g der Verbindung der Formel (II-1) in Xylol/Methanol (0,500 mol, ca. 31 %ig) wird vorgelegt. Methanol wird bei Normaldruck abdestilliert. 105 g Na-Methanolat in Methanol (0,583 mol, 30 %ig) werden in 1 h bei 85 °C dosiert. Während der Na-Methanolat Dosierung wird Methanol bei 85 °C abdestilliert. Nach Ende der Dosierung wird 4 h bei 85 °C nachgerührt. Um die Temperatur von 85 °C zu halten, wird zwischendurch Methanol destilliert. Nach 4 h ist der Umsatz vollständig. Das Reaktionsgemisch wird für die weitere Umsetzung auf 70 °C abgekühlt.

Zu dieser Mischung (Verbindung der Formel (III-1) in Xylol/Methanol (34 %ig, 0,495 mol)) werden 118,1 g HCl 2 % ig (0,06 mol) zugegeben. Das Gemisch wird 15 min. bei 70 °C nachgerührt und anschließend im Vakuum bei 350-250 mbar und 50-70 °C methanolfrei destilliert. Der Sumpf ist zweiphasig. Es wird auf 50 °C abgekühlt und 150 g Xylol und 15,3 g Triethylamin (0,150 mol) zugegeben. Anschließend werden 76,74 g Chloressigsäureethylester (0,700 mol, 99 %ig) in 2 h bei 50 °C dosiert. Durch die Paralleldosierung von Natronlauge 32 %ig wird der pH-Wert zwischen 9,5-10 gehalten. Es wird ½ h bei 50 °C nachgerührt. Mit einer 18 %igen Salzsäurelösung wird ein pH-Wert von 2 eingestellt und Wasser zugegeben. Für die Phasentrennung wird auf 75 °C aufgeheizt, wobei der pH-Wert mit Natronlauge nachgestellt wird und anschließend die Phasen getrennt. Es werden 150 g Natriumhydrogencarbonat-Lösung 2 %ig bei 75 °C zugegeben und die Phasen getrennt. Der pH-Wert liegt bei ca. 8. Die organische Phase wird bei ca. 75 mbar und 60-70 °C entwässert und auf konzentriert. 90,6 g Methylcyclohexan werden bei 75 °C zugegeben, wobei Feststoff (Verbindung der Formel (I-1)) ausfällt. Das Reaktionsgemisch wird auf 109 °C (Rückfluss) aufgeheizt, auf 20 °C abgekühlt und isoliert. Der Feststoff (Verbindung der Formel (I-1)) wird durch eine Verdrängungswäsche mit MCH gewaschen und anschließend getrocknet.

Die isolierte Ausbeute beträgt 96-98 % d. Th. bezogen auf die Verbindung der Formel (II-1).

### Beispiel 5: Herstellung der Verbindung der Formel (I-1)

537,8 g der Verbindung der Formel (II-1) in Xylol/Methanol (0,500 mol, ca. 31 %ig) wird vorgelegt. Methanol wird bei Normaldruck abdestilliert. 105 g Na-Methanolat in Methanol (0,583 mol, 30 %ig) werden in 1 h bei 85 °C dosiert. Während der Na-Methanolat Dosierung wird Methanol bei 85 °C abdestilliert. Nach Ende der Dosierung wird 4 h bei 85 °C nachgerührt. Um die Temperatur von 85 °C zu halten, wird zwischendurch Methanol destilliert. Nach 4 h ist der Umsatz vollständig. Das Reaktionsgemisch wird für die weitere Umsetzung auf 80 °C abgekühlt.

80 g Wasser werden bei Raumtemperatur vorgelegt. Zu dieser Vorlage werden 437,8 g der Verbindung der Formel (III-1) in Xylol/Methanol (34 %ig, 0,495 mol) zugegeben. Das Gemisch wird 15 min. bei 70 °C nachgerührt und anschließend im Vakuum bei 350-250 mbar und 50-70 °C methanolfrei destilliert. Es wird auf 50 °C abgekühlt und 150 g Xylol und 15,3 g Triethylamin (0,150 mol) zugegeben. Anschließend werden 76,74 g Chloressigsäureethylester (0,700 mol, 99 %ig) in 2 h bei 50 °C dosiert. Durch die Paralleldosierung von Natronlauge 32 %ig wird der pH-Wert zwischen 9,5-10 gehalten. Es wird ½ h bei 50 °C nachgerührt. Mit einer 18 %igen Salzsäurelösung wird ein pH-Wert von 2 eingestellt und Wasser zugegeben. Für die Phasentrennung wird auf 75 °C aufgeheizt, wobei der pH-Wert mit Natronlauge nachgestellt wird und anschließend die Phasen getrennt. Es werden 150 g Natriumhydrogencarbonat-Lösung 2 %ig bei 75 °C zugegeben und die Phasen getrennt. Der pH-Wert liegt bei ca. 8.

Die organische Phase wird bei ca. 75 mbar und 60-70 °C entwässert und aufkonzentriert. 90,6 g Methylcyclohexan werden bei 75 °C zugegeben, wobei Feststoff (Verbindung der Formel (I-1)) ausfällt. Das Reaktionsgemisch wird auf 109 °C (Rückfluss) aufgeheizt, auf 20 °C abgekühlt und isoliert. Der Feststoff (Verbindung der Formel (I-1)) wird durch eine Verdrängungswäsche mit MCH gewaschen und anschließend getrocknet.

Die isolierte Ausbeute beträgt 96-98 % d. Th. bezogen auf die Verbindung der Formel (II-1).

### Beispiel 6: Herstellung der Verbindung der Formel (I-1)

537,8 g der Verbindung der Formel (II-1) in Xylol/Methanol (0,500 mol, ca. 31 %ig) wird vorgelegt. Methanol wird bei Normaldruck abdestilliert.105 g Na-Methanolat in Methanol (0,583 mol, 30 %ig) werden in 1 h bei 85 °C dosiert. Während der Na-Methanolat Dosierung wird Methanol bei 85 °C abdestilliert. Nach Ende der Dosierung wird 4 h bei 85 °C nachgerührt. Um die Temperatur von 85 °C zu halten, wird zwischendurch Methanol destilliert. Nach 4 h ist der Umsatz vollständig. Das Reaktionsgemisch wird für die weitere Umsetzung auf 80 °C abgekühlt.

Zu dieser Reaktionsmischung (437,8 g der Verbindung der Formel (III-1) in Xylol/Methanol (34 %ig, 0,495 mol)) werden 80 g Wasser zugegeben (es entsteht MeOH und NaCl). Das Gemisch wird 15 min. bei 70 °C nachgerührt und anschließend im Vakuum bei 350-250 mbar und 50-70 °C methanolfrei destilliert. Der Sumpf ist zweiphasig. Es wird auf 50 °C abgekühlt und 150 g Xylol und 15,3 g Triethylamin (0,150 mol) zugegeben. Anschließend werden 76,74 g Chloressigsäureethylester (0,700 mol, 99 %ig) in 2 h bei 50 °C dosiert. Durch die Paralleldosierung von Natronlauge 32 %ig wird der pH-Wert zwischen 9,5-10 gehalten Es wird ½ h bei 50 °C nachgerührt. Mit einer 18 %igen Salzsäurelösung wird ein pH-Wert von 2 eingestellt und Wasser zugegeben. Für die Phasentrennung wird auf 75 °C aufgeheizt, wobei der pH-Wert mit Natronlauge nachgestellt wird und anschließend die Phasen getrennt.

Es werden 150 g Natriumhydrogencarbonat-Lösung 2 %ig bei 75 °C zugegeben und die Phasen getrennt. Der pH-Wert liegt bei ca. 8.

Die organische Phase wird bei ca. 75 mbar und 60-70 °C entwässert und aufkonzentriert. 90,6 g Methylcyclohexan werden bei 75 °C zugegeben, wobei Feststoff (Verbindung der Formel (I-1)) ausfällt. Das Reaktionsgemisch wird auf 109 °C (Rückfluss) aufgeheizt, auf 20 °C abgekühlt und isoliert. Der Feststoff (Verbindung der Formel (I-1)) wird durch eine Verdrängungswäsche mit MCH gewaschen und anschließend getrocknet.

Ausbeute: 94 % d. Th. bezogen auf die Verbindung der Formel (II-1).

### Beispiel 7: Herstellung der Verbindung der Formel (I-1)

537,8 g der Verbindung der Formel (II-1) in Xylol/Methanol (0,500 mol, ca. 31 %ig) wird vorgelegt. Methanol wird bei Normaldruck abdestilliert. 20,69 g Na-Methanolat in Methanol (0,15 mol, 98 %ig) werden in 1 h bei 85 °C dosiert. Während der Dosierung wird Methanol bei 85 °C abdestilliert. Nach Ende der Dosierung wird 4 h bei 85 °C nachgerührt. Um die Temperatur von 85 °C zu halten, wird zwischendurch Methanol destilliert. Nach 4 h ist der Umsatz vollständig. Das Reaktionsgemisch wird für die weitere Umsetzung auf 80 °C abgekühlt.

118,1 g HCl 2 % ig (0,06 mol) werden bei Raumtemperatur vorgelegt. Zu dieser Vorlage werden 437,8 g der Verbindung der Formel (III-1) in Xylol/Methanol (34 %ig, 0,495 mol) zugegeben (es entsteht MeOH und NaCl). Das Gemisch wird 15 min. bei 70 °C nachgerührt und anschließend im Vakuum bei 350-250 mbar und 50-70 °C methanolfrei destilliert. Es wird auf 50 °C abgekühlt und 150 g Xylol und 20,69 g Dimethylbenzylamin (0,150 mol) zugegeben. Anschließend werden 76,74 g Chloressigsäureethylester (0,700 mol, 99 %ig) in 2 h bei 50 °C dosiert. Durch die Paralleldosierung von Natronlauge 32 %ig wird der pH-Wert zwischen 9,5-10 gehalten. Es wird ½ h bei 50 °C nachgerührt. Mit einer 18 %igen Salzsäurelösung wird ein pH-Wert von 2 eingestellt und Wasser zugegeben. Für die Phasentrennung wird auf 75 °C aufgeheizt, wobei der pH-Wert mit Natronlauge nachgestellt wird und anschließend die Phasen getrennt.

Es werden 150 g Natriumhydrogencarbonat-Lösung 2 %ig bei 75 °C zugegeben und die Phasen getrennt. Der pH-Wert liegt bei ca. 8. Die organische Phase wird bei ca. 75 mbar und 60-70 °C entwässert und aufkonzentriert. 90,6 g Methylcyclohexan werden bei 75 °C zugegeben, wobei Feststoff (Verbindung der Formel (I)) ausfällt. Das Reaktionsgemisch wird auf 109 °C (Rückfluss) aufgeheizt, auf 20 °C abgekühlt und isoliert. Der Feststoff (Verbindung der Formel (I-1)) wird durch eine Verdrängungswäsche mit MCH gewaschen und anschließend getrocknet.

Ausbeute: 83 % d. Th. bezogen auf die Verbindung der Formel (II-1).

### Beispiel 8: Herstellung der Verbindung der Formel (I-1)

537,8 g der Verbindung der Formel (II-1) in Xylol/Methanol (0,500 mol, ca. 31 %ig) wird vorgelegt. Methanol wird bei Normaldruck abdestilliert. 105 g Na-Methanolat in Methanol (0,583 mol, 30 %ig) werden in 1 h bei 85 °C dosiert. Während der Na-Methanolat Dosierung wird Methanol bei 85 °C abdestilliert. Nach Ende der Dosierung wird 4 h bei 85 °C nachgerührt. Um die Temperatur von 85 °C zu halten, wird zwischendurch Methanol destilliert. Nach 4 h ist der Umsatz vollständig. Das Reaktionsgemisch wird für die Umsetzung zum Wirkstoff auf 80 °C abgekühlt.

118,1 g HCl 2 % ig (0,06 mol) werden bei Raumtemperatur vorgelegt. Zu dieser Vorlage werden 437,8 g der Verbindung der Formel (III-1) in Xylol/Methanol (34 %ig, 0,495 mol) zugegeben. Das Gemisch wird 15 min. bei 70 °C nachgerührt und anschließend im Vakuum bei 350-250 mbar und 50-70 °C methanolfrei destilliert. Es wird auf 50 °C abgekühlt und 150 g Xylol und 15,3 g Triethylamin (0,150 mol) zugegeben. Anschließend werden 76,74 g Chloressigsäureethylester (0,700 mol, 99 %ig) in 2 h bei 50 °C dosiert. Durch die Paralleldosierung von Natronlauge 32 %ig wird der pH-Wert zwischen 9,5-10 gehalten. Es wird ½ h bei 50 °C nachgerührt. Mit einer 18 %igen Salzsäurelösung wird ein pH-Wert von 2 eingestellt und Wasser zugegeben. Für die Phasentrennung wird auf 75 °C aufgeheizt, wobei der pH-Wert mit Natronlauge nachgestellt wird und anschließend die Phasen getrennt.

Es werden 150 g Natriumhydrogencarbonat-Lösung 2 %ig bei 75 °C zugegeben und die Phasen getrennt. Der pH-Wert liegt bei ca. 8.

Die organische Phase wird bei ca. 75 mbar und 60-70 °C entwässert. Das Reaktionsgemisch wird auf 109 °C (Rückfluss) aufgeheizt, auf 0 °C abgekühlt und isoliert. Der Feststoff (Verbindung der Formel (I-1)) wird durch eine Verdrängungswäsche mit Xylol gewaschen und anschließend getrocknet.

Ausbeute: 95 % d. Th. bezogen auf die Verbindung der Formel (II-1).

### Beispiel 9: Herstellung der Verbindung der Formel (I-1)

537,8 g der Verbindung der Formel (II-1) in Xylol/Methanol (0,500 mol, ca. 31 %ig) wird vorgelegt. Methanol wird bei Normaldruck abdestilliert. 105 g Na-Methanolat in Methanol (0,583 mol, 30 %ig) werden in 1 h bei 85 °C dosiert. Während der Na-Methanolat Dosierung wird Methanol bei 85 °C abdestilliert. Nach Ende der Dosierung wird 4 h bei 85 °C nachgerührt. Um die Temperatur von 85 °C zu halten, wird zwischendurch Methanol destilliert. Nach 4 h ist der Umsatz vollständig. Das Reaktionsgemisch wird für die weitere Umsetzung auf 80 °C abgekühlt.

118,1 g HCl 2 % ig (0,06 mol) werden bei Raumtemperatur vorgelegt. Zu dieser Vorlage werden 437,8 g der Verbindung der Formel (III-1) in Xylol/Methanol (34 %ig, 0,495 mol) zugegeben. Das Gemisch wird 15 min. bei 70 °C nachgerührt und anschließend im Vakuum bei 350-250 mbar und 50-70 °C methanolfrei destilliert. Es wird auf 50 °C abgekühlt und 150 g Xylol und 4,06 g Aliqat336 (0,01 mol) zugegeben. Anschließend werden 76,74 g Chloressigsäureethylester (0,700 mol, 99 %ig) in 2 h bei 50 °C dosiert. Durch die Paralleldosierung von Natronlauge 32 %ig wird der pH-Wert zwischen 9,5-10 gehalten. Es wird ½ h bei 50 °C nachgerührt. Mit einer 18 %igen Salzsäurelösung wird ein pH-Wert von 2 eingestellt und Wasser zugegeben. Für die Phasentrennung wird auf 75 °C aufgeheizt, wobei der pH-Wert mit Natronlauge nachgestellt wird und anschließend die Phasen getrennt.

Es werden 150 g Natriumhydrogencarbonat-Lösung 2 %ig bei 75 °C zugegeben und die Phasen getrennt. Der pH-Wert liegt bei ca. 8.

Die organische Phase wird bei ca. 75 mbar und 60-70 °C entwässert und aufkonzentriert. 90,6 g Methylcyclohexan werden bei 75 °C zugegeben, wobei Feststoff (Verbindung der Formel (I-1)) ausfällt. Das Reaktionsgemisch wird auf 109 °C (Rückfluss) aufgeheizt, auf 20 °C abgekühlt und isoliert. Der Feststoff (Verbindung de Formel (I-1)) wird durch eine Verdrängungswäsche mit MCH gewaschen und anschließend getrocknet.

Ausbeute: 85 % d. Th. bezogen auf die Verbindung der Formel (II-1).

### Beispiel 10: Herstellung der Verbindung der Formel (I-1)

537,8 g der Verbindung der Formel (II-1) in Xylol/Methanol (0,500 mol, ca. 31 %ig) wird vorgelegt. Methanol wird bei Normaldruck abdestilliert. 105 g Na-Methanolat in Methanol (0,583 mol, 30 %ig) werden in 1 h bei 85 °C dosiert. Während der Na-Methanolat Dosierung wird Methanol bei 85 °C abdestilliert. Nach Ende der Dosierung wird 4 h bei 85 °C nachgerührt. Um die Temperatur von 85 °C zu halten, wird zwischendurch Methanol destilliert. Nach 4 h ist der Umsatz vollständig. Das Reaktionsgemisch wird für die weitere Umsetzung auf 80 °C abgekühlt.

118,1 g HCl 2 % ig (0,06 mol) werden bei Raumtemperatur vorgelegt. Zu dieser Vorlage werden 437,8 g der Verbindung der Formel (III-1) in Xylol/Methanol (34 %ig, 0,495 mol) zugegeben. Das Gemisch wird 15 min. bei 70 °C nachgerührt und anschließend im Vakuum bei 350-250 mbar und 50-70 °C methanolfrei destilliert. Es wird auf 50 °C abgekühlt und 150 g Xylol, 6,12 g Triethylamin (0,150 mol) und 4,06 g Aliqat336 (0,01 mol) zugegeben. Anschließend werden 76,74 g Chloressigsäureethylester (0,700 mol, 99 %ig) in 2 h bei 50 °C dosiert. Durch die Paralleldosierung von Natronlauge 32 %ig wird der pH-Wert zwischen 9,5-10 gehalten. Es wird ½ h bei 50 °C nachgerührt. Mit einer 18 %igen Salzsäurelösung wird ein pH-Wert von 2 eingestellt und Wasser zugegeben. Für die Phasentrennung wird auf 75 °C aufgeheizt, wobei der pH-Wert mit Natronlauge nachgestellt wird und anschließend die Phasen getrennt.

Es werden 150 g Natriumhydrogencarbonat-Lösung 2 %ig bei 75 °C zugegeben und die Phasen getrennt. Der pH-Wert liegt bei ca. 8.

Die organische Phase wird bei ca. 75 mbar und 60-70 °C entwässert und aufkonzentriert. 90,6 g Methylcyclohexan werden bei 75 °C zugegeben, wobei Feststoff (Verbindung der Formel (I-1)) ausfällt. Das Reaktionsgemisch wird auf 109 °C (Rückfluss) aufgeheizt, auf 20 °C abgekühlt und isoliert. Der Feststoff (Verbindung der Formel (I-1)) wird durch eine Verdrängungswäsche mit MCH gewaschen und anschließend getrocknet.

Ausbeute: 86 % d. Th. bezogen auf die Verbindung der Formel (II-1).

### Beispiel 11: Herstellung der Verbindung der Formel (I-1)

537,8 g der Verbindung der Formel (II-1) in Xylol/Methanol (0,500 mol, ca. 31 %ig) wird vorgelegt. Methanol wird bei Normaldruck abdestilliert. 105 g Na-Methanolat in Methanol (0,583 mol, 30 %ig) werden in 1 h bei 85 °C dosiert. Während der Na-Methanolat Dosierung wird Methanol bei 85 °C abdestilliert. Nach Ende der Dosierung wird 4 h bei 85 °C nachgerührt. Um die Temperatur von 85 °C zu halten, wird zwischendurch Methanol destilliert. Nach 4 h ist der Umsatz vollständig. Das Reaktionsgemisch wird auf 60 °C abgekühlt und Wasser zugegeben. Die Phasen werden getrennt und die organische Phase wird entsorgt.

Die wässrige Enolphase wird im Vakuum bei 350-250 mbar und 50-70 °C methanolfrei destilliert. Es wird auf 65 °C abgekühlt, Salzsäure zugegeben und anschließend festes Natriumhydrogencarbonat zugegeben. In diese Mischung werden 67,11 g Chloressigsäureethylester (0,600 mol, 97 %ig) in 2 h bei 65 °C dosiert. Der Ansatz wird 3 Stunden bei 75 °C nachgerührt. Ein Umsatz (Verbindung der Formel (I-1)) von 85 % (HPLC) wird bestimmt.

### Beispiel 12: Herstellung der Verbindung der Formel (I-1)

537,8 g der Verbindung der Formel (II-1) in Xylol/Methanol (0,500 mol, ca. 31 %ig) wird vorgelegt. Methanol wird bei Normaldruck abdestilliert. 105 g Na-Methanolat in Methanol (0,583 mol, 30 %ig) werden in 1 h bei 85 °C dosiert. Während der Na-Methanolat Dosierung wird Methanol bei 85 °C abdestilliert. Nach Ende der Dosierung wird 4 h bei 85 °C nachgerührt. Um die Temperatur von 85 °C zu halten, wird zwischendurch Methanol destilliert. Nach 4 h ist der Umsatz vollständig. Das Reaktionsgemisch wird auf 60 °C abgekühlt und Wasser zugegeben. Die Phasen werden getrennt und die organische Phase wird entsorgt.

Die wässrige Enolphase wird im Vakuum bei 350-250 mbar und 50-70 °C methanolfrei destilliert. Es wird auf 65 °C abgekühlt, Salzsäure zugegeben und anschließend festes Natriumhydrogencarbonat eingetragen. In diese Mischung werden 10 g (0,098 mol) Triethylamin und 89,48 g Chloressigsäureethylester (0,800 mol, 97 %ig) in 2 h bei 65 °C dosiert. Der Ansatz wird 3 Stunden bei 75 °C nachgerührt. Ein Umsatz (Verbindung der Formel (I-1)) von 75 % (HPLC) wird bestimmt.

### Beispiel 13: Herstellung der Verbindung der Formel (I-1)

537,8 g der Verbindung der Formel (II-1) in Xylol/Methanol (0,500 mol, ca. 31 %ig) wird vorgelegt. Methanol wird bei Normaldruck abdestilliert. 105 g Na-Methanolat in Methanol (0,583 mol, 30 %ig) werden in 1 h bei 85 °C dosiert. Während der Na-Methanolat Dosierung wird Methanol bei 85 °C abdestilliert. Nach Ende der Dosierung wird 4 h bei 85 °C nachgerührt. Um die Temperatur von 85 °C zu halten, wird zwischendurch Methanol destilliert. Nach 4 h ist der Umsatz vollständig. Das Reaktionsgemisch wird auf 60 °C abgekühlt und Wasser zugegeben. Die Phasen werden getrennt und die organische Phase wird entsorgt.

Die wässrige Enolphase wird im Vakuum bei 350-250 mbar und 50-70 °C methanolfrei destilliert. Es wird auf 55 °C abgekühlt, festes Natriumhydrogencarbonat eingetragen. In diese Mischung werden 72,70 g Chloressigsäureethylester (0,650 mol, 97 %ig) in 2 h bei 65 °C dosiert. Der Ansatz wird 3 Stunden bei 55 °C nachgerührt und alkoholfrei destilliert. Es werden 150 ml Xylol zugeben und auf 80 °C erwärmt. Zwecks Kristallisation wird auf 20 °C abgekühlt und filtriert. Ein Umsatz (Verbindung der Formel (I-1)) von ca. 79 % wird im HPLC nachgewiesen.

### Vergleichsbeispiel: Verfahren zur Herstellung von Verbindung der Formel (I-1) mit Zwischenisolierung von Verbindung der Formel (III-1)

Es werden 807,69 g einer Lösung der Verbindung der Formel (II-1) (0,81 mol) in DMAC vorgelegt. Bei einer Innentemperatur von 60 - 65 °C werden 159,04 g einer 30%igen Natriummethylatlösung in Methanol (0,88 mol) in ca. 2,5 h zudosiert. Anschließend wird Methanol im Vakuum abdestilliert.

Anschließend werden 440 g Wasser zugegeben und bei 60 °C mit 60,3 g konz. Salzsäure (37 %) auf pH 5,5 angesäuert. Der ausgefallene Feststoff wird bei 20 °C abgesaugt, 2 mal mit je 130 g Wasser gewaschen und im Vakuum getrocknet. Ausbeute 159,9 g Feststoff mit einem Gehalt von 98,0 g der Verbindung (III-1) entsprechend einer Ausbeute von 94,5 %.

Zu einer Mischung aus 500 g Methylcyclohexan, 28,4 g Triethylamin (98%) und 76,9 g der Verbindung der Formel (III-1) (98,0 %) werden unter Siedebedingungen in 15 Min. 30,4 g Chlorameisensäureethylester (98 %) zudosiert. Nach 4 h Nachrühren unter Rückfluss wird der Ansatz auf 80 °C abgekühlt und mit 180 g Wasser versetzt. Die wässrige Phase mit dem gelösten Triethylamin Hydrochlorid wird abgetrennt. Aus der organischen Phase wird das Restwasser durch azeotrope Destillation entfernt und zur vollständigen Kristallisation des Wirkstoffes auf 10 °C abgekühlt.

Der ausgefallene Feststoff wird abgesaugt, mit 100 g Methylcyclohexan gewaschen und im Vakuum getrocknet.

Ausbeute: 88,6 g mit einem Gehalt von 98,8 % Spirotetramat (Verbindung der Formel (I-1)) entsprechend 93,4 % Ausbeute.

Die Ausbeute über zwei Stufen bezogen auf die Verbindung der Formel (II-1) beträgt 88,3 % d.Th..

## Patentansprüche

1. Verfahren zur Herstellung von Verbindungen der Formel (I) 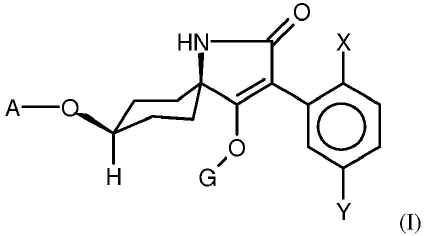 in welcher
X für Methyl steht,
Y für Methyl steht,
A für Methyl steht,
G für die Gruppe 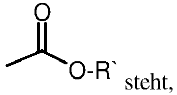
worin
R' für Ethyl steht,
**dadurch gekennzeichnet, dass** man in einer Eintopfreaktion zunächst Verbindungen der Formel (II) 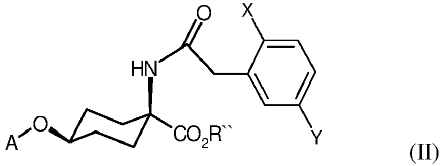 in welcher X, Y und A die oben genannten Bedeutungen haben und
R" für Methyl steht,
in Gegenwart von Natriummethylat und in Gegenwart von DMAC zu Verbindungen der Formel (III) 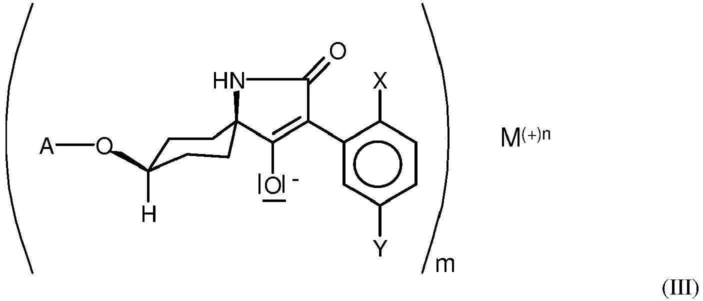
in welcher X, Y und A die oben genannten Bedeutungen haben und
M für Natrium steht,
m für die Zahl 1 steht und n für die Zahl 1 steht
cyclisiert
und mit Verbindungen der Formel (IV) 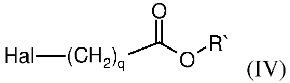
in welcher
R' für Ethyl steht,
q für die Zahl 0 oder 1 steht,
und Hal für Chlor steht,
gegebenenfalls in Gegenwart von DMAC und gegebenenfalls in Gegenwart von Trietylamin als Säurebindemittel und gegebenenfalls in Anwesenheit von Aliquat 336 als Phasentransferkatalysator umsetzt, wobei die bei einer Rückspaltung aus Verbindungen der Formel (I) entstandenen Verbindungen der Formel (III) durch Umsetzung mit Verbindungen der Formel (IV) gegebenenfalls in Gegenwart von Xylol und gegebenenfalls in Gegenwart von Trietyhlamin als Säurebindemittel zu Verbindungen der Formel (I) rückgeführt werden.

2. Verfahren zur Herstellung von Verbindungen der Formel (I) 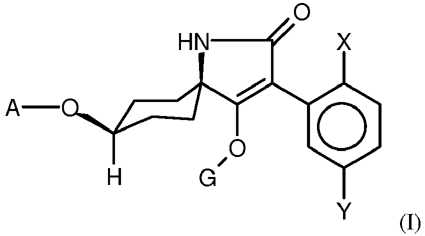
in welcher X, Y, A und G die oben angegebenen Bedeutungen haben.
**dadurch gekennzeichnet, dass** man in einer Eintopfreaktion zunächst Verbindungen der Formel (II) 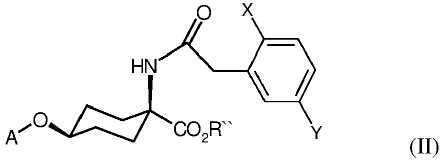
in welcher X, Y, A und R"die oben genannten Bedeutungen haben,
in Gegenwart von Natriummethylat und in Gegenwart von Xylol zu Verbindungen der Formel (III) 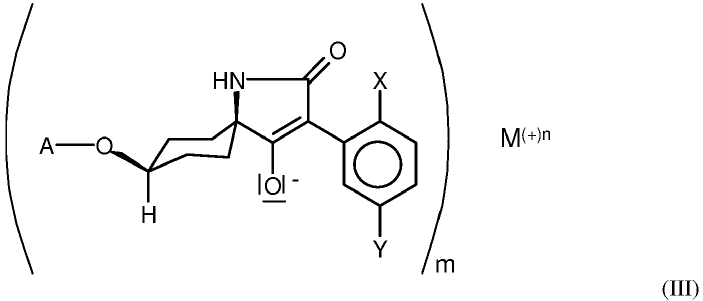
in welcher X, Y, A, M, m und n die oben genannten Bedeutungen haben
cyclisiert
und mit Verbindungen der Formel (IV) 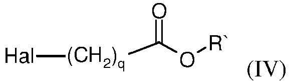
in welcher
R', q und Hal die oben genannten Bedeutungen haben,
gegebenenfalls in Gegenwart von Xylol und gegebenenfalls in Gegenwart von Trietylamin als Säurebindemittel und gegebenenfalls in Anwesenheit von Aliquat 336 als Phasentransferkatalysator umsetzt

3. Verfahren gemäß Anspruch 2, wobei die Umsetzung der Verbindungen der Formel (III) mit Verbindungen der Formel (IV) in Gegenwart von Wasser durchgeführt wird.

4. Verfahren gemäß Anspruch 3, wobei für die Umsetzung der Verbindungen der Formel (II) zu Verbindungen der Formel (III) als Co-Lösungsmittel Methanol eingesetzt wird.

5. Verfahren gemäß Anspruch 3, wobei als Base für die Einstellung des pH-Werts wässrige Natronlauge verwendet wird.

6. Verfahren zur Herstellung von Verbindungen der Formel (I) 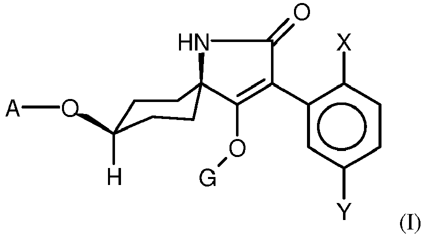
in welcher X, Y, A und G die oben angegebenen Bedeutungen haben.
**dadurch gekennzeichnet, dass** man in einer Eintopfreaktion zunächst Verbindungen der Formel (II) 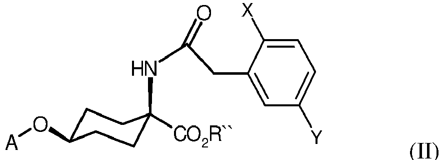
in welcher X, Y, A und R"die oben genannten Bedeutungen haben,
in Gegenwart von Natriummethylat und in Gegenwart von DMAC zu Verbindungen der Formel (III) 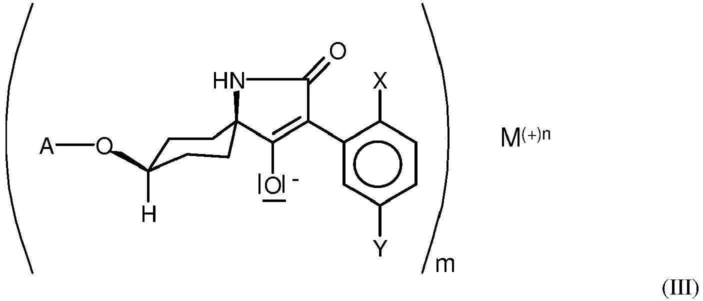
in welcher X, Y, A, M, m und n die oben genannten Bedeutungen haben
cyclisiert
und mit Verbindungen der Formel (IV) 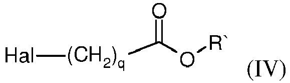
in welcher
R', q und Hal die oben genannten Bedeutungen haben,
gegebenenfalls in Gegenwart von DMAC und gegebenenfalls in Gegenwart von Trietylamin als Säurebindemittel und gegebenenfalls in Anwesenheit von Aliquat 336 als Phasentransferkatalysator umsetzt.

## Claims

1. Method for preparing compounds of the formula (I) 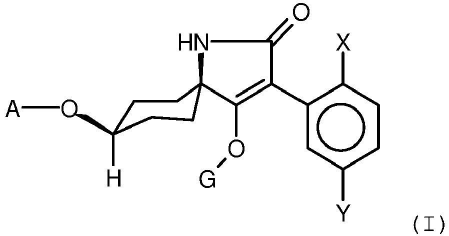 in which
X is methyl,
Y is methyl,
A is methyl,
G is the group 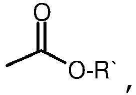
in which
R' is ethyl,
**characterized in that** firstly compounds of the formula (II) 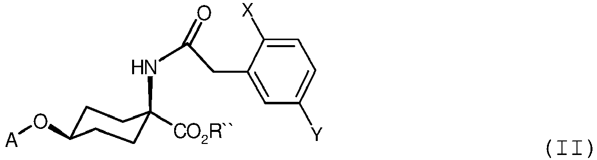 in which X, Y and A are as defined above and
R" is methyl,
are cyclized in a one-pot reaction in the presence of sodium methoxide and in the presence of DMAC to give compounds of the formula (III) 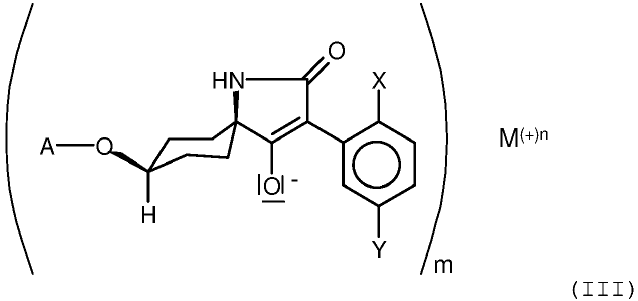 in which X, Y and A are as defined above and
M is sodium,
m is the number 1 and n is the number 1
and are reacted with compounds of the formula (IV) 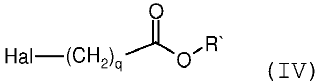 in which
R' is ethyl,
q is the number 0 or 1,
and Hal is chlorine,
optionally in the presence of DMAC and optionally in the presence of triethylamine as acid binder and optionally in the presence of Aliquat 336 as phase transfer catalyst, wherein the compounds of the formula (III) formed in a redissociation of compounds of the formula (I), by reaction with compounds of the formula (IV) optionally in the presence of xylene and optionally in the presence of triethylamine as acid binder, are recycled to give compounds of the formula (I).

2. Method for preparing compounds of the formula (I) 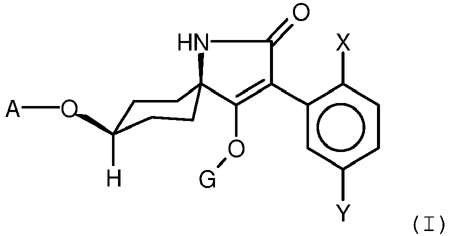
in which X, Y, A and G are as defined above,
**characterized in that** firstly compounds of the formula (II) 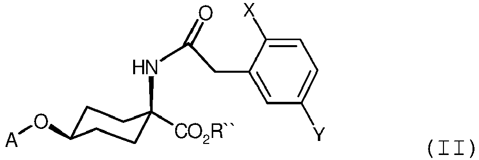
in which X, Y, A and R" are as defined above,
are cyclized in a one-pot reaction in the presence of sodium methoxide and in the presence of xylene to give compounds of the formula (III) 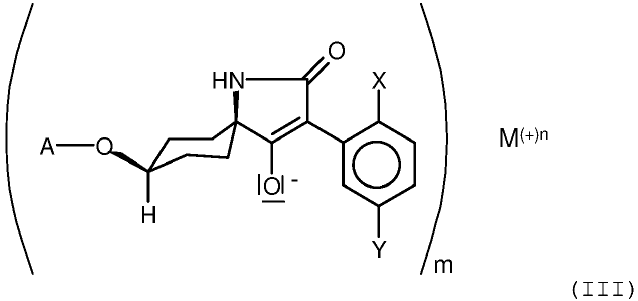
in which X, Y, A, M, m and n are as defined above
and are reacted with compounds of the formula (IV) 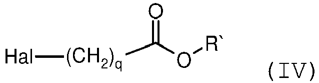
in which
R', q and Hal are as defined above,
optionally in the presence of xylene and optionally in the presence of triethylamine as acid binder and optionally in the presence of Aliquat 336 as phase transfer catalyst.

3. Method according to Claim 2, wherein the reaction of the compounds of the formula (III) with compounds of the formula (IV) is carried out in the presence of water.

4. Method according to Claim 3, wherein methanol is used as co-solvent for the reaction of the compounds of the formula (II) to give compounds of the formula (III).

5. Method according to Claim 3, wherein aqueous sodium hydroxide solution is used as base for adjusting the pH.

6. Method for preparing compounds of the formula (I) 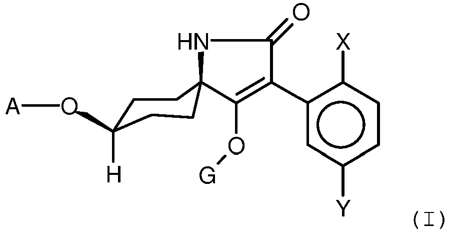
in which X, Y, A and G are as defined above,
**characterized in that** firstly compounds of the formula (II) 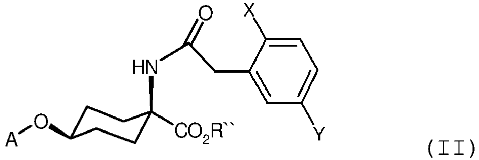
in which X, Y, A and R" are as defined above,
are cyclized in a one-pot reaction in the presence of sodium methoxide and in the presence of DMAC to give compounds of the formula (III) 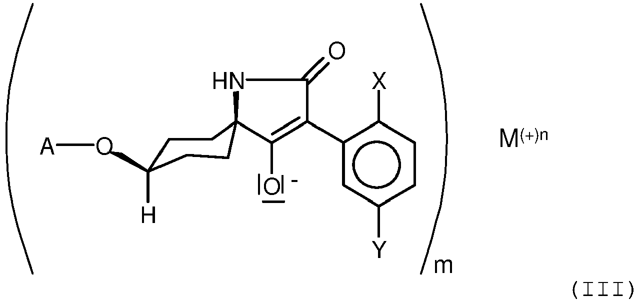
in which X, Y, A, M, m and n are as defined above
and are reacted with compounds of the formula (IV) 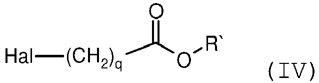
in which
R', q and Hal are as defined above,
optionally in the presence of DMAC and optionally in the presence of triethylamine as acid binder and optionally in the presence of Aliquat 336 as phase transfer catalyst.

## Revendications

1. Procédé de préparation de composés de formule (I) 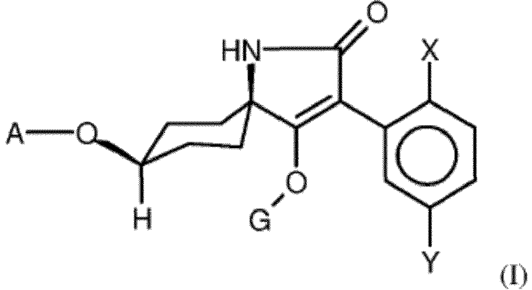 dans laquelle
X représente méthyle,
Y représente méthyle,
A représente méthyle,
G représente le groupe 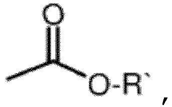
dans lequel
R' représente éthyle,
**caractérisé en ce que**, dans une réaction dans un seul récipient, on cyclise tout d'abord des composés de Formule (II) 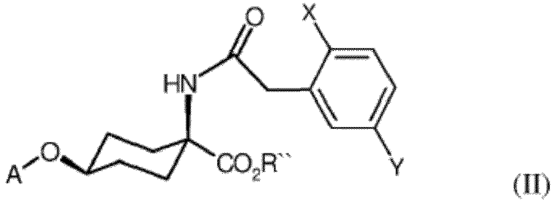
dans laquelle X, Y et A possèdent les significations mentionnées ci-dessus et
R" représente méthyle,
en présence de méthylate de sodium et en présence de DMAC pour donner des composés de formule (III) 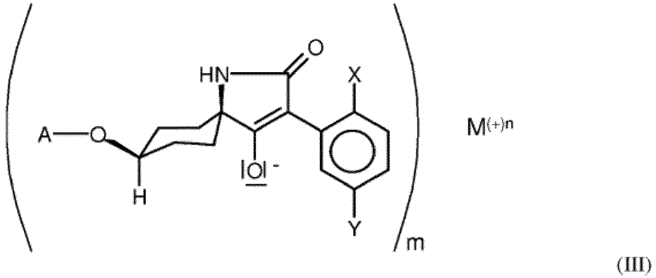
dans laquelle X, Y et A possèdent les significations mentionnées ci-dessus et
M représente sodium,
m représente le nombre 1 et n représente le nombre 1
et on met à réagir avec des composés de formule (IV) 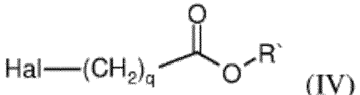
dans laquelle
R' représente éthyle,
q représente le nombre 0 ou 1,
et Hal représente chlore,
éventuellement en présence de DMAC, éventuellement en présence de triéthylamine en tant que liant d'acide et éventuellement en présence d'Aliquat 336 en tant que catalyseur de transfert de phases, les composés de formule (III) produits lors d'une redécomposition de composés de formule (I) étant recyclés par transformation avec des composés de formule (IV) éventuellement en présence de xylène et éventuellement en présence de triéthylamine en tant que liant d'acide pour donner des composés de formule (I).

2. Procédé de préparation de composés de formule (I) 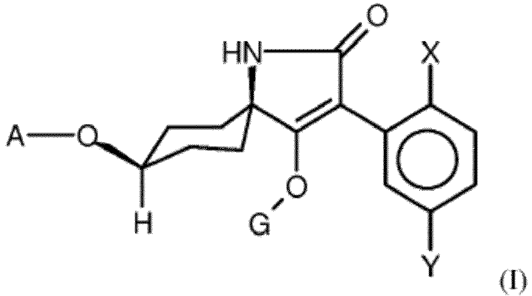
dans laquelle X, Y, A et G possèdent les significations indiquées ci-dessus,
**caractérisé en ce qu'**on cyclise tout d'abord, dans une réaction dans un seul récipient, des composés de formule (II) 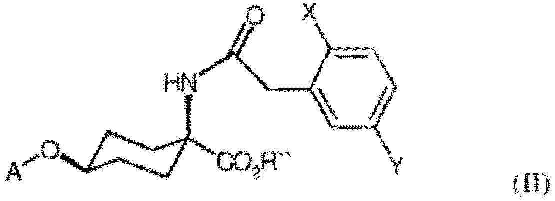
dans laquelle X, Y, A et R" possèdent les significations mentionnées ci-dessus,
en présence de méthylate de sodium et en présence de xylène pour donner des composés de formule (III) 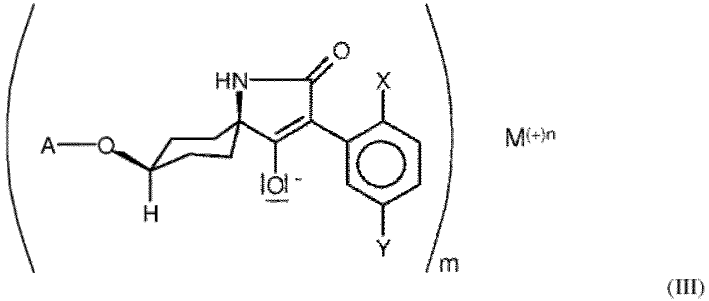
dans laquelle X, Y, A, M, m et n possèdent les significations mentionnées ci-dessus
et on met à réagir avec des composés de formule (IV) 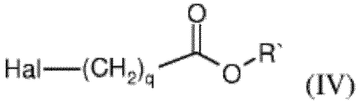
dans laquelle
R', q et Hal possèdent les significations mentionnées ci-dessus,
éventuellement en présence de xylène et éventuellement en présence de triéthylamine en tant que liant d'acide et éventuellement en présence d'Aliquat 336 en tant que catalyseur de transfert de phases.

3. Procédé selon la revendication 2, la transformation des composés de formule (III) avec des composés de formule (IV) étant réalisée en présence d'eau.

4. Procédé selon la revendication 3, dans lequel pour la transformation des composés de formule (II) en composés de formule (III), du méthanol est utilisé en tant que cosolvant.

5. Procédé selon la revendication 3, de la soude caustique aqueuse étant utilisée en tant que base pour l'ajustement de la valeur du pH.

6. Procédé de préparation de composés de formule (I) 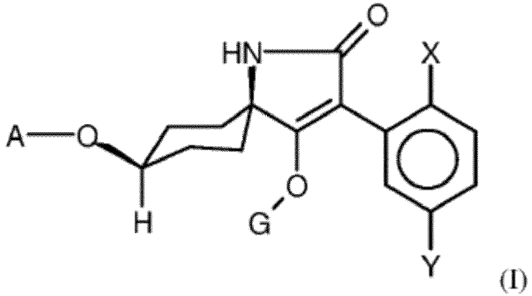
dans laquelle X, Y, A et G possèdent les significations indiquées ci-dessus,
**caractérisé en ce qu'**on cyclise, dans une réaction dans un seul récipient, tout d'abord des composés de formule (II) 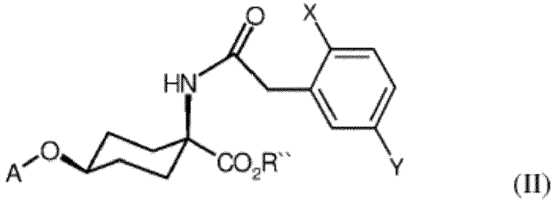
dans laquelle X, Y, A et R" possèdent les significations mentionnées ci-dessus,
en présence de méthylate de sodium et en présence de DMAC pour donner des composés de formule (III) 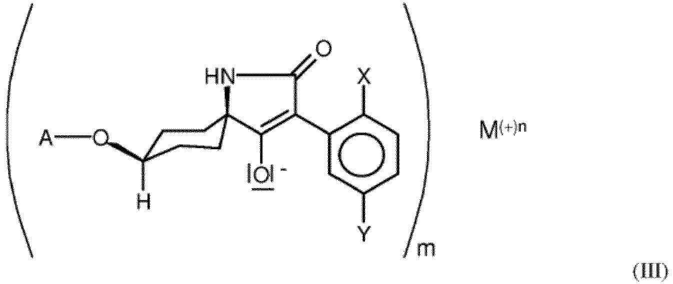
dans laquelle X, Y, A, M, m et n possèdent les significations mentionnées ci-dessus
et on met à réagir avec des composés de formule (IV) 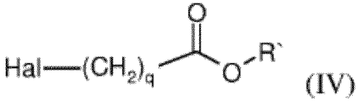
dans laquelle
R', q et Hal possèdent les significations mentionnées ci-dessus,
éventuellement en présence de DMAC et éventuellement en présence de triéthylamine en tant que liant d'acide et éventuellement en présence d'Aliquat 336 en tant que catalyseur de transfert de phases.
